# EUROPÄISCHE PATENTSCHRIFT

(11) **EP 2 377 461 B1**
(45) Veröffentlichungstag und Bekanntmachung des Hinweises auf die Patenterteilung: **24.08.2016**
(21) Anmeldenummer: 11159333.1
(22) Anmeldetag: 23.03.2011
(51) Int. Cl.: A61N 1/375, A61N 1/372, A61B 5/07

(54) **Mini-Implantat**
Mini implant
Mini-implant

(30) Priorität: 13.04.2010 US 323371 P
(43) Veröffentlichungstag der Anmeldung: 19.10.2011
(73) Patentinhaber: BIOTRONIK SE & Co. KG, 12359 Berlin (DE)
(72) Erfinder: Diebold, Michael, 12169, Berlin (DE)
(74) Vertreter: Galander, Marcus

(56) Entgegenhaltungen:
- US-A1- 2002 138 009
- US-A1- 2004 260 391
- US-A1- 2008 102 096
- US-A1- 2008 234 598
- US-A1- 2008 319 280

## Beschreibung

Die Erfindung betrifft ein Mini-Implantat, welches aufgrund seiner geringen Abmessungen leicht in den Körper eines Patienten eingeführt werden kann.

Bekannt sind beispielsweise Mini-Implantate als einfache Identifikationsimplantate, die eine einfache RFID-Schaltung aufweisen (Radio Frequency Identification) und dazu beispielsweise eine Spule und einen Ferritkern sowie eine vorgelagerte Elektronik besitzen. Ein solches Implantat ist z.B. in US 2008/0102096 A1 offenbart. Die RFID-Schaltung ist beispielsweise von Glas ummantelt. Das Glas bildet dann ein biokompatibles Gehäuse.

US 2008/0234598 A1 beschreibt einen implantierbaren sensor gemäß erstem Teil des Anspruchs 1. Dieser sensor client zur Identifizierung einer kontraiktalen Kondition eines Patienten.

US 2004/0260391 A1 offenbart einen Stent für eine kontrollierte Freisetzung eines Medikaments. Die Kappe des Medikamentenreservoirs kann sich thermisch bedingt auflösen oder für das Medikament durchlässig werden.

Ziel der vorliegenden Erfindung ist es, die Integrationsdichte von Mini-Implantaten zu erhöhen, um entweder komplexere Funktionalitäten realisieren zu können oder Mini-Implantate noch wesentlich zu verkleinern. Ziel sind beispielsweise RFID-Implantate von der Größe eines Reiskorns mit integrierter Sensorik.

Erfindungsgemäß wird dieses Ziel mit einem Mini-Implantat erreicht, welches ein Gehäuse aus biokompatiblem, elektrisch isolierendem Kunststoff besitzt sowie eine elektrische Schaltung, die von elektrischen und/oder elektronischen Komponenten gebildet ist. Außerdem weist das Mini-Implantat elektrisch leitende Leiterbahnen zur elektrischen Verbindung der elektrischen und/oder elektronischen Komponenten miteinander auf. Erfindungsgemäß sind diese Leiterbahnen wenigstens zum Teil auf Innenflächen des Gehäuses angeordnet und besitzen eine biokompatible Oberfläche.

Die elektrischen oder elektronischen Komponenten sind wenigstens zum Teil im Inneren des Gehäuses angeordnet und mit den Leiterbahnen verbunden.

Indem die Leiterbahnen direkt auf Oberflächen des Gehäuses aufgebracht sind, dient das Gehäuse quasi als "Leiterplatte" für die vom Gehäuse beherbergte elektrische oder elektronische Schaltung. Auf diese Weise ist es möglich, auf engerem Raum mehr Komponenten unterzubringen, um so die Gesamtgröße des Implantats zu verkleinern oder bei gleichbleibender Größe komplexere Funktionen zu realisieren.

Gemäß einer bevorzugten Ausführungsvariante ist der biokompatible, elektrisch isolierende Kunststoff des Gehäuses ein Flüssigkristallpolymer (LCP: Liquid Crystal Polymer). Flüssigkristallpolymer ist biokompatibel und elektrisch isolierend und daher für ein hermetisch dichtes Gehäuse eines erfindungsgemäßen Implantats besonders geeignet.

Die Leiterbahnen bestehen vorzugsweise aus Gold oder einem anderen biokompatiblen Leiter oder sind wenigstens auf ihrer Außenseite mit Gold beschichtet, so dass die Leiterbahnen wenigstens auf ihrer Außenseite selbst auch biokompatibel sind.

Die elektrische Schaltung bildet gemäß einer bevorzugten Ausführungsvariante wenigstens eine RFID-Einheit und kann darüber hinaus oder alternativ auch als Steuereinheit oder Sensorschaltung ausgebildet sein.

Insbesondere im letztgenannten Fall ist es vorteilhaft, wenn wenigstens eine der Leiterbahnen sich bis auf eine Außenseite des Gehäuses erstreckt, um dort beispielsweise als Sensor-Elektrode zu fungieren.

Gemäß weiterer Ausführungsvarianten des Mini-Implantats besitzt das Gehäuse nach außen offene oder verschlossene Kavitäten, die chemische Wirkstoffe enthalten können, die beispielsweise therapeutische Wirkung haben können oder der Analyse im Rahmen einer verbrauchenden Sensorik dienen. Derartige Kavitäten können so ausgebildet sein, dass diese zunächst verschlossen sind und sich erst nach Implantation des Implantats, beispielsweise durch Wärmeeinwirkung oder Biodegradation oder einen anderen chemischen oder physikalischen Effekt zeitlich oder individuell gesteuert, öffnen. Hierzu sind die Kavitäten vorzugsweise mit einer Schicht aus biodegradierbarem oder unter Wärmeeinfluss schmelzendem Material verschlossen.

Erfindungsgemäß hat das Gehäuse des Mini-Implantats eine längliche Form mit zwei Längsenden und weist wenigstens eine Kappe an wenigstens einem der Längsenden auf. Dabei ist diese Kappe als Membran ausgebildet und erlaubt so beispielsweise eine dosierte Wirkstoffabgabe oder auch das Eindringen von Körperflüssigkeit, um so einen Kontakt zwischen einer hinter der Kappe angeordneten Sensoranordnung und einer zu analysierenden Körperflüssigkeit herzustellen.

Bei den Varianten des Implantats, deren elektrische Komponenten wenigstens teilweise und zeitweise mit Körperflüssigkeit in Kontakt stehen müssen, wie beispielsweise entsprechende Sensoren, ist erfindungsgemäß eine fluiddichte Durchführung vorgesehen, über die eine jeweils außenliegende elektrische Komponente mit den im Inneren des Gehäuses liegenden elektrischen Komponenten verbunden ist.

Vorzugsweise weist das Mini-Implantat wenigstens eine fluiddichte Kammer auf, in der sich solche Komponenten der elektrischen oder elektronischen Schaltung befinden, die möglichst vor äußeren Einflüssen zu schützen sind.

Gemäß einer weiteren bevorzugten Ausführungsvariante ist das Gehäuse des Mini-Implantats mit einem biokompatiblen Material beschichtet, wobei das Material beispielsweise so gewählt ist, dass sich ein gewünschtes Einwachsverhalten des Implantats nach Implantation ergibt.

Anstelle eines Gehäuses aus einem Thermoplast, wie beispielsweise Flüssigkristallpolymer, kann das Gehäuse auch aus einem Duroplast geformt sein. Im zweiten Fall ist jedoch eine nachträgliche Verformung des Gehäuses unter Zuhilfenahme von Wärme nur schwer oder gar nicht möglich.

Die Erfindung soll nun anhand eines Ausführungsbeispiels in Bezug auf die Figuren näher erläutert werden. Von den Figuren zeigt:
- Fig. 1:: Ein erfindungsgemäßes Mini-Implantat in der Außenansicht;
- Fig. 2:: ein erfindungsgemäßes Mini-Implantat in einer ersten Ausführungsvariante im Längsschnitt; und
- Fig. 3:: eine zweite Variante eines erfindungsgemäßen Mini-Implantats im Längsschnitt.

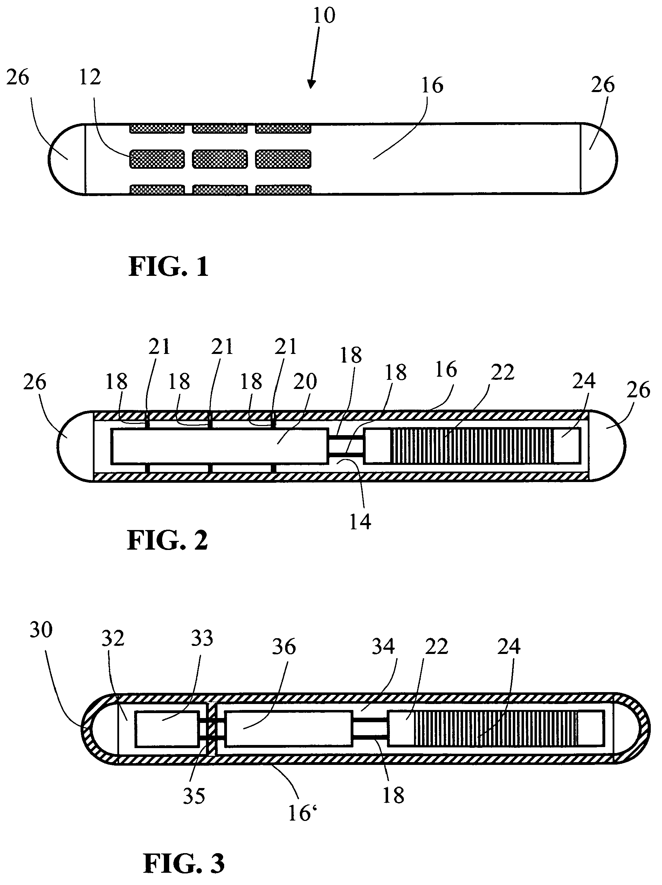

Fig. 1 zeigt ein erfindungsgemäßes Mini-Implantat 10 mit auf der Außenseite des Mini-Implantats angebrachten Sensoren 12. Die Sensoren können beispielsweise einfache elektrisch leitende Oberflächen sein, alternativ aber beispielsweise auch elektrochemische Sensoren.

Fig. 2 zeigt einen Längsschnitt durch das Mini-Implantat 10 aus Fig. 1. Zu erkennen sind auf der Innenfläche 14 eines Gehäuses 16 des Mini-Implantats 10 angeordnete Leiterbahnen 18, die der Kontaktierung einer elektronischen Schaltung 20 in Form eines Mikrochips dienen. Außerdem kontaktieren die Leiterbahnen 18 über entsprechende, nicht näher dargestellte, hermetisch dichte Durchführungen 21 die Sensoren 12 auf der Außenseite des Gehäuses 16 des Implantats 10.

Die Leiterbahnen 18 bestehen im bevorzugten Ausführungsbeispiel aus Gold und sind somit selbst biokompatibel. Alternativ können die Leiterbahnen auch von auf der Außenseite mit Gold beschichteten Kupferbahnen gebildet sein.

Das Gehäuse 16 des Mini-Implantats 10 ist vorzugsweise von einem biokompatiblen, elektrisch nicht leitenden Thermoplast, insbesondere von Flüssigkristallpolymer (LCP), gebildet.

Im Gehäuse 16 des Implantats 10 ist vorzugsweise auch eine Spule 22 mit einem Ferritkern 24 angeordnet, über die auf induktivem Wege Energie in das Mini-Implantat 10 eingespeist werden kann und die außerdem der Übertragung von Identifikationsdaten und anderen Daten dient, wie grundsätzlich bekannt ist.

Das Gehäuse 16 ist an seinen beiden Längsenden mit Endkappen 26 verschlossen. Bei der in Fig. 2 abgebildeten Ausführungsvariante sind diese Endkappen 26 hermetisch dicht.

Erfindungsgemäß ist eine der Endkappen oder auch beide der Endkappen als für Körperflüssigkeit durchlässige Membran ausgeführt. Dies ist bei der in Fig. 3 abgebildeten Ausführungsvariante bei der linken Endkappe 30 der Fall. Diese verschließt eine Kavität 32, die als Analysekammer dient, in der sich beispielsweise ein Drucksensor oder ein elektrochemischer Sensor 33 befindet. Die Kavität 32 ist von einer hermetisch geschlossenen Kammer 34 in dem Gehäuse 16' durch eine Trennwand mit Durchführung 35 gas- und flüssigkeitsdicht getrennt. In der Kammer 34 befindet sich eine Mikroelektronik 36 als Steuerelektronik sowie die bereits aus der Ausführungsvariante aus Fig. 2 bekannte Spule 22 mit ihrem Ferritkern 24.

Bestandteile des Gehäuses 16 oder 16' können beispielsweise leicht im Spritzgussverfahren hergestellt werden und nach Einbau der Komponenten des Implantats 10 durch Ultraschallschweißen miteinander dicht verbunden werden.

Es versteht sich, dass das Gehäuse auch noch weitere Kammern oder Kavitäten und weitere an sich bekannte Komponenten aufweisen kann, so dass auf Basis der erfindungsgemäßen Grundgedanken auch komplexere Mini-Implantate zu realisieren sind.

### Bezugszeichenliste

- 10: Mini-Implantat
- 12: Sensoren
- 14: Innenfläche
- 16,16': Gehäuse
- 18: Leiterbahnen
- 20: elektronische Schaltung
- 21: hermetisch dichte Durchführung
- 22: Spule
- 24: Ferritkern
- 26: Endkappen
- 30: linke Endkappe
- 32: Kavität
- 33: Sensor
- 34: Kammer
- 35: Trennwand mit Durchführung
- 36: Mikroelektronik

## Patentansprüche

1. Mini-Implantat (10) mit
einem Gehäuse (16, 16') aus biokompatiblem, elektrisch isolierendem Kunststoff,
einer elektrischen Schaltung (20), die elektrische oder elektronische Komponenten oder beides aufweist,
einem elektrischen oder elektro-chemischen Sensor (12, 33), der mit der elektrischen Schaltung (20) verbunden ist und
elektrisch leitenden Leiterbahnen (18) mit biokompatibler Oberfläche,
von denen die Leiterbahnen (18) wenigstens zum Teil auf Innenflächen des Gehäuses (16, 16') angeordnet sind und Komponenten der elektrischen Schaltung (20, 36) im Inneren des Gehäuses (16, 16') angeordnet und mit den Leiterbahnen (18) verbunden sind, wobei der elektrische oder elektro-chemische Sensor (12, 33) in einer Analysekammer des Implantats (10) angeordnet und über eine fluiddichte Durchführung (21) elektrisch mit der Schaltung (20, 36) verbunden ist,
**dadurch gekennzeichnet, dass** Gehäuse (16, 16') eine längliche Form mit zwei Längsenden hat und wenigstens eine Kappe (26, 30) an wenigstens einem Längsende ausweist, wobei die Kappe (26, 30) als Membran ausgebildet ist, die eine Analysekammer (34) des Mini-Implantats (10) verschließt und das Eindringen von Körperflüssigkeiten erlaubt.

2. Mini-Implantat (10) nach Anspruch 1, **dadurch gekennzeichnet, dass** die elektrische Schaltung (20, 36) eine RFID-Einheit bildet.

3. Mini-Implantat (10) nach Anspruch 1 oder 2, **dadurch gekennzeichnet, dass** die elektrische Schaltung (20, 36) eine Steuereinheit bildet.

4. Mini-Implantat (10) nach einem der Ansprüche 1 bis 3, **dadurch gekennzeichnet, dass** die Leiterbahnen (18) sich wenigstens teilweise bis auf eine Außenseite des Gehäuses (16, 16') erstrecken.

5. Mini-Implantat (10) nach einem der Ansprüche 1 bis 4, **dadurch gekennzeichnet, dass** das Gehäuse (16, 16') wenigstens eine zunächst geschlossene, zu öffnende Kavitäten (32) aufweist.

6. Mini-Implantat (10) nach Anspruch 5, **dadurch gekennzeichnet, dass** die Kavität (32) mittels wenigstens einer Schicht aus biodegradierbarem Material geschlossen ist, welche die Eigenschaft hat, nach Implantation des Implantats durch Biodegradation abgebaut zu werden, so dass sich Kavität (32) nach Implantation öffnet.

7. Mini-Implantat (10) nach Anspruch 5, **dadurch gekennzeichnet, dass** die Kavität (32) mittels wenigstens einer unter Wärmeeinfluss schmelzenden Schicht geschlossen ist.

8. Mini-Implantat (10) nach einem der Ansprüche 5 bis 7, **dadurch gekennzeichnet, dass** die Kavität (32) mit einem Wirkstoff gefüllt ist.

9. Mini-Implantat (10) nach einem der Ansprüche 1 bis 8, **dadurch gekennzeichnet, dass** der Kunststoff des Gehäuses (16, 16') ein Thermoplast, bevorzugt ein Flüssigkristall-Polymer, ist.

10. Mini-Implantat (10) nach einem der Ansprüche 1 bis 9, **dadurch gekennzeichnet, dass** die elektrische Schaltung (20, 36) eine Telemetrieeinheit umfasst, die dazu ausgebildet, Daten vom Implantat (10) drahtlos auszusenden.

11. Mini-Implantat (10) nach einem der Ansprüche 1 bis 10, **dadurch gekennzeichnet, dass** das Gehäuse (16, 16') auf seiner Außenseite eine Beschichtung trägt.

## Claims

1. A mini implant (10) having
a housing (16, 16') made of biocompatible, electrically insulating plastic,
an electric circuit (20), which comprises electric or electronic components or both,
an electric or electrochemical sensor (12, 33), which is connected to the electric circuit (20), and
electrically conductive tracks (18) having a biocompatible surface,
at least some of the tracks (18) being disposed on inner surfaces of the housing (16, 16') and components of the electric circuit (20, 36) being disposed inside the housing (16, 16') and connected to the tracks (18), wherein the electric or electrochemical sensor (12, 33) is disposed in an analysis chamber of the implant (10) and is electrically connected to the circuit (20, 36) via a fluid-tight feedthrough (21),
**characterised in that** the housing (16, 16') has an elongate form with two longitudinal ends and comprises at least one cap (26, 30) at least at one longitudinal end, wherein the cap (26, 30) is formed as a membrane, which closes an analysis chamber (34) of the mini implant (10) and allows the infiltration of bodily fluids.

2. The mini implant (10) according to claim 1, **characterised in that** the electric circuit (20, 36) forms an RFID unit.

3. The mini implant (10) according to claim 1 or 2, **characterised in that** the electric circuit (20, 36) forms a control unit.

4. The mini implant (10) according to any one of claims 1 to 3, **characterised in that** the tracks (18) extend at least in part as far as an outer surface of the housing (16, 16').

5. The mini implant (10) according to any one of claims 1 to 4, **characterised in that** the housing (16, 16') comprises at least one initially closed cavity (32), which is/are to be opened.

6. The mini implant (10) according to claim 5, **characterised in that** the cavity (32) is closed by means of at least one layer made of biodegradable material, which layer has the property of being decomposed by biodegradation after implantation of the implant so that the cavity (32) opens after implantation.

7. The mini implant (10) according to claim 5, **characterised in that** the cavity (32) is closed by means of at least one layer which melts under the influence of heat.

8. The mini implant (10) according to any one of claims 5 to 7, **characterised in that** the cavity (32) is filled with an active agent.

9. The mini implant (10) according to any one of claims 1 to 8, **characterised in that** the plastic of the housing (16, 16') is a thermoplastic, preferably a liquid-crystal polymer.

10. The mini implant (10) according to any one of claims 1 to 9, **characterised in that** the electric circuit (20, 36) comprises a telemetry unit, which is designed to wirelessly transmit data from the implant (10).

11. The mini implant (10) according to any one of claims 1 to 10, **characterised in that** the housing (16, 16') carries a coating on its outer surface.

## Revendications

1. Mini-implant (10) avec
un boîtier (16, 16') à base d'une matière synthétique biocompatible, isolante électriquement,
un circuit électrique (20) qui présente des composants électriques, ou électroniques, ou les deux,
un capteur électrique ou électrochimique (12, 33) qui est relié avec le circuit électrique (20) et
des pistes conductrices (18) électriquement avec une surface biocompatible,
parmi lesquels les pistes conductrices (18) sont au moins en partie disposées sur des surfaces intérieures du boîtier (16, 16') et des composants du circuit électrique (20, 36) sont disposés à l'intérieur du boîtier (16, 16') et sont reliés avec les pistes conductrices (18), où le capteur électrique ou électrochimique (12, 33) est disposé dans une chambre d'analyse de l'implant (10) et est relié électriquement avec le circuit (30, 36) par l'intermédiaire d'une traversée (21) étanche aux fluides,
**caractérisé en ce que** le boîtier (16, 16') a une forme allongée avec deux extrémités longitudinales et au moins un capuchon (26, 30) à au moins une extrémité longitudinale, où le capuchon (26, 30) est conçu sous la forme d'une membrane, qui ferme une chambre d'analyse (34) du mini-implant (10) et qui permet l'entrée de liquides corporels.

2. Mini-implant (10) selon la revendication 1, **caractérisé en ce que** le circuit électrique (20, 36) forme une unité RFID.

3. Mini-implant (10) selon la revendication 1, ou 2, **caractérisé en ce que** le circuit électrique (20, 36) forme une unité de commande.

4. Mini-implant (10) selon l'une des revendications précédentes, **caractérisé en ce que** les pistes conductrices (18) s'étendent au moins partiellement jusqu'au côté extérieur du boîtier (16, 16').

5. Mini-implant (10) selon l'une des revendications 1 à 4, **caractérisé en ce que** le boîtier (16, 16') présente au moins une cavité (32) d'abord fermée, puis à ouvrir.

6. Mini-implant (10) selon la revendication 5, **caractérisé en ce que** la cavité (32) est fermée au moyen d'au moins une couche de matériau biodégradable, laquelle a la propriété de pouvoir être dégradée par biodégradation après l'implantation de l'implant, de sorte que la cavité (32) s'ouvre après l'implantation.

7. Mini-implant (10) selon la revendication 5, **caractérisé en ce que** la cavité (32) est fermée au moyen d'au moins une couche fondant sous l'influence de la chaleur.

8. Mini-implant (10) selon l'une des revendications 5 à 7, **caractérisé en ce que** la cavité (32) est remplie avec une matière active.

9. Mini-implant (10) selon l'une des revendications 1 à 8, **caractérisé en ce que** la matière synthétique du boîtier (16, 16') est un thermoplastique, de préférence un polymère à cristaux liquides.

10. Mini-implant (10) selon l'une des revendications 1 à 9, **caractérisé en ce que** le circuit électrique (20, 36) comprend une unité de télémétrie qui est conçue pour émettre sans fil des données de l'implant (10).

11. Mini-implant (10) selon l'une des revendications 1 à 10, **caractérisé en ce que** le boîtier (16, 16') porte un revêtement sur sa face extérieure.
